# EUROPÄISCHE PATENTSCHRIFT

(11) **EP 0 592 989 B1**
(45) Veröffentlichungstag und Bekanntmachung des Hinweises auf die Patenterteilung: **08.05.2002**
(21) Anmeldenummer: 93116446.1
(22) Anmeldetag: 11.10.1993
(51) Int. Cl.: C07K 14/00, B01J 20/32, C08B 37/00, B01D 15/08

(54) **Verfahren zur quantitativen selektiven Entfernung oder präperativen Gewinnung von Tumor-Nekrose-Faktor (TNF) und Lipopolysacchariden (LPS) aus wässrigen Flüssigkeiten**
Process of the selective and quantitative removal or preparation of tumor necrosis factor (TNF) and lipopolysaccharides (LPS) of aqueous solutions
Procédé d'élimination sélective et quantitative ou préparation du facteur de nécrose tumorale (TNF) et de lipopolysaccharides (LPS) de solution aqueuse

(30) Priorität: 12.10.1992 DE 4234363; 15.09.1993 DE 4331358
(43) Veröffentlichungstag der Anmeldung: 20.04.1994
(73) Patentinhaber: B. BRAUN MELSUNGEN AG, 34212 Melsungen (DE)
(72) Erfinder: Boos, Karl-Siegfried, Prof. Dr., D-82131 Gauting (DE); Seidel, Dietrich, Prof. Dr., D-82340 Feldafing (DE); von der Haar, Friedrich, Prof. Dr., D-34212 Melsungen (DE)
(74) Vertreter: Böhm, Brigitte, Dipl.-Chem. Dr.

(56) Entgegenhaltungen:
- EP-A- 0 143 369
- EP-A- 0 180 766
- EP-A- 0 209 786
- EP-A- 0 225 867
- EP-A- 0 263 518
- EP-A- 0 431 593
- WO-A-91/01808
- DE-A- 3 421 731
- GB-A- 2 092 470
- PATENT ABSTRACTS OF JAPAN vol. 013 no. 211 (C-597) ,17.Mai 1989 & JP-A-01 029399 (DENKI KAGAKU KOGYO KK) 31.Januar 1989,
- DATABASE WPI Section Ch, Week 8618 Derwent Publications Ltd., London, GB; Class A96, AN 86-115980 & JP-A-61 056 133 ( KAGAKU-OYOBI-KESSEI) , 20.März 1986

## Beschreibung

Die Erfindung betrifft die Verwendung eines Adsorptionsmaterials aus porösen Trägermaterialien mit kovalent gebundenen funktionellen Gruppen aus synthetischen, halbsynthetischen und/oder natürlichen linearen und/oder verzweigten Polyanionenketten zur Entfernung des Tumor-Nekrose-Faktors (TNF) und Lipophilsacchariden (LPS) aus Blut, Plasma oder Serum.

Die selektive Eliminierung des TNF und von LPS aus Humanblut und/oder Humanplasma ist aus medizinischer Sicht insbesondere für die Behandlung des septischen Schocks erwünscht.

Der septische Schock entsteht als Komplikation bei Infektioen des Menschen durch gram-negative Bakterien. Die Prognose dieses Krankheitsbildes ist unter der derzeitigen Standardtherapie schlecht. Bei bis zu 50 % der Patienten mit septischem Schock muß trotz aller therapeutischen Bemühungen mit einem letalen Ausgang gerechnet werden. In den USA wird die Anzahl der durch einen septischen Schock hervorgerufenen Todesfälle auf ca. 100000 pro Jahr geschätzt (Parillo, J.E. "Septic Shock in Humans" in: Annals of Internal Medicine, Vol. 113, No. 3, 1990, 227-242). Vom intensiv-therapeutischen Standpunkt ist daher eine selektive extracorporale Elimination dieses Pathogens wünschenswert, insbesondere auch deshalb, weil beispielsweise die Gabe von hochwirksamen Antibiotika oder von Immunglobulinen sowie eine Plasmaaustauschbehandlung die Prognose nicht wesentlich verbessern.

Die Pathogenese des septischen Schocks beginnt mit der Invasion gram-negativer Bakterien in die Blutbahn. Die Infektion führt beim Zerfall der Bakterien zur Freisetzung von Lipopolysacchariden (LPS) aus der äußeren Bakterienmembran. Diese sogenannten Endotoxine aktivieren die Makrophagen und führen zur Sekretion des Tumor-Nekrose-Faktors (TNF). Die LPS-Moleküle besitzen eine stäbchenartige Form und sind aus drei strukturell unterschiedlichen Regionen aufgebaut. Der Träger der toxischen Eigenschaften ist das Lipid-A. Diese Subregion mit einem Molekulargewicht von 2000 Dalton besteht aus einem phosphorylierten D-Glucosamin-Disaccharid, an das ester- und amidartig mehrere langkettige Fettsäuren gebunden sind.

Der Tumor-Nekrose-Faktor ist ein Polypeptid (157 Aminosäuren, Molekulargewicht: 17 x 10³ Dalton) und zählt als Hormon des Immunsystems zu der Klasse der Cytokine. Der TNF nimmt unter diesen Polypeptidmediatoren eine Schlüsselrolle hinsichtlich der Pathogenese des septischen Schocks ein. So besteht bei Patienten mit beispielsweise einer Meningokokken-Sepsis eine Korrelation zwischen der TNF-Konzentration im Plasma und dem Grad der septischen Schock-Symptome bzw. dem späteren Todeseintritt (Grau, G.E. et al., Immunol. Rev. 112, 1989, 49 ff.). Erhöhte TNF-Plasmakonzentrationen finden sich auch bei Patienten mit parasitären Erkrankungen und anderen Infektionen (Scuderi, P. et al., Lancet II, 1986, 1229 ff.). Das klinische Bild der Sepsis korreliert auch in den meisten Fällen mit dem Verlauf und der Höhe der Endotoxinkonzentration im Blut (Nitsche, D. et al., Intensive Care Med. 12 Suppl., 1986, 185 ff.).

Der Erfindung liegt deshalb die Aufgabe zugrunde, eine neue Verwendung eines Adsorptionsmittels zur selektiven Eliminierung von TNF und LPS aus Vollblut, Plasma und/oder Serum bereitzustellen, die zugleich die Voraussetzung zur einfachen und sicheren Anwendung in einem extracorporalen Perfusionssystem für den Menschen erfüllt.

Verständlicherweise gilt die Nutzung dieser dafür verwendeten Materialien in vitro für analytische und/oder präparative Belange.

Um eine derartige Verwendung ex vivo zu medizinisch-therapeutischen Zwecken nutzen zu können, müssen u.a. folgende Voraussetzungen erfüllt sein:
1) Die Elimination des Pathogens sollte möglichst selektiv erfolgen.
2) Das Eliminationsverfahren darf keine physiologischen Schutzmechanismen, wie beispielsweise das Komplementoder Gerinnungssystem, aktivieren.
3) Die Bindungskapazität des Adsorbens sollte optimalen praktischen Anforderungen genügen.
4) Das Adsorbens muß mit Hitze oder gamma-Strahlen sterilisierbar sein.
5) Das Adsorbens sollte nur eine minimale Freisetzung von Partikeln im Mikrometerbereich aufweisen.
6) Das Adsorbens sollte eine ausreichend hohe Durchflußgeschwindigkeit im Bereich bis zu 200 ml/min erlauben.

Es sind bereits Materialien für die Abtrennung von LDL oder VLDL aus Blut bekannt, die jedoch nicht auf Polyanionen basieren. So beschreibt GB 2 092 470 ein Material mit der Struktur R-A-X, wobei dieses Material keine Polyanionen darstellt.

EP 0 292 786 beschreibt einen klassischen Kationenaustauscher, mit Hilfe dessen gentechnologisch hergestellter TNF gereinigt werden kann. Es handelt sich nicht um ein Polyanion. Auch ist über eine Verwendbarkeit zur Abtrennung von LPS nichts ausgesagt.

JP-B-630 029 36 befaßt sich mit einem Affinitätschromatorgaphiematerial aus einem Cellulosesulfatester oder einem quervernetzten Polysaccharidsulfatester. Ein Hinweis auf eine Möglichkeit zur Behandlung von an Sespis leidenden Patienten ist nicht gegeben.

Erfindungsgemäß wurde die genannte Aufgabe gelöst durch die Verwendung eines Adsorptionsmaterials aus einem porösen Trägermaterial mit einem mittleren Porendurchmesser von kleiner 30 nm oder/und mit einer molekularen Ausschlußgrenze für globuläre Proteine von kleiner 10⁶, insbesondere von kleiner 2 x 10⁴ Dalton mit daran kovalent gebundenen funktionellen Gruppen aus synthetischen oder/und halbsynthetischen oder/und natürlichen Polyanionenketten mit einem mittleren Molekulargewicht von 600 bis 10⁶ Dalton, insbesondere 5 x 10³ bis 5 x 10⁵ Dalton, und zwar in linearer oder verzweigter Form zur Herstellung eines Mittels zur Behandlung eines septischen Schockes unter quantitativer, selektiver Entfernung von Tumor-Nekrose-Faktor (TNF) und Lipopolysacchariden aus Blut, Plasma oder Serum.

Die bei der erfindungsgemäßen Verwendung bevorzugten natürlichen Polyanionen, die mit Hilfe dem Fachmann bekannter Verfahren kovalent an eine entsprechende Trägermatrix gebunden werden, sind biologische Polycarbon- und/oder Polysulfonsäuren, wie beispielsweise sulfatierte Polysaccharide (Heparin, Dextransulfat, Chondroitinsulfat etc.) Die erfindungsgemäß bevorzugten synthetischen bzw. halbsynthetischen Polyanionen, die mit Hilfe dem Fachmann bekannter Verfahren kovalent an eine entsprechende Trägermatrix gebunden werden, sind Polymerisate oder Copolymerisate folgender Monomeren:
1) Vinyl-Typ, wie beispielsweise Acrylsäure, Methacrylsäure, Vinylsulfonsäure, Maleinsäure etc.
2) Acrylsäure- und/oder Methacrylsäurederivate der Formel H₂C = CR₁ - CO - R₂, wobei der Substituent R₁ Wasserstoff oder eine Methylgruppe und R₂ eine amid- oder esterartig verknüpfte lineare und/oder verzweigtkettige aliphatische Sulfonsäure, Carbonsäure- und/oder Phosphorsäuregruppe ist.
3) Styrol-Typ, wie beispielsweise Styrolsulfonsäure, Anetholsulfonsäure, Styrolphosphorsäure etc.
4) Peptid-Typ, wie beispielsweise Glutaminsäure, Asparaginsäure etc.
5) Nucleinsäure-Typ, wie beispielsweise Adenosin-3',5'diphosphat, Guanosin-3',5'-diphosphat etc.

Die bei der erfindungsgemäßen Verwendung verwendete Trägermatrix kann aus porösem Glas, porösem und/oder mit organischen Polymerisaten oder Copolymerisaten beschichtetem Kieselgel, vernetzten Kohlenhydraten, wie beispielsweise mikrogranulare oder regenerierte und/oder derivatisierte Cellulose, Dextranen und Agarose und/oder aus organischen Polymerisaten oder Copolymerisaten bestehen.

Adsorbentien, die die aufgeführten Eigenschaften aufweisen, sind bereits beschrieben, wie beispielsweise in EP 0 143 369 (Synthetische Polyanionen immobilisiert auf Kieselgel), EP 0 110 409 und EP 0 225 867 (Poröse und/oder derivatisierte Cellulosegele, an die synthetische oder natürliche Polyanionen kovalent gebunden sind), US 4 103 685 (Natürliche Anionen gebunden an Agarose-Beads), EP-Anmeldung 83 112 042 (Natürliche Polyanionen immobilisiert auf organischen Copolymeren), DE 39 26 539 (Tentakel-Kationenaustauscher auf Copolymer-Basis), EP 0 424 698 (Polyacrylsäure immobilisiert auf einem organischen Copolymer) und DE 36 17 672 (Natürliche und synthetische Polyanionen gebunden an Kieselgel).

Die zuvor beschriebenen Adsorbentien werden bevorzugt zur selektiven Adsorption von Low-Density-Lipoproteinen (LDL), Very-Low-Density-Lipoproteinen (vLDL) und/oder Fibrinogen hergestellt und verwendet.

Es erwies sich jedoch unerwarteterweise, daß derartige Adsorbentien auch den Tumor-Nekrose-Faktor (Beispiel 1) und Lipopolysaccharide (Beispiel 2) binden.

Die mit derartigen Adsorptionsmaterialien erzielten Erfolge sind jedoch für die Belange einer selektiven Elimination von TNF und LPS in einem extracorporalen Perfusionssystem zu medizinisch-therapeutischen Zwecken unbefriedigend, da die Bindungskapazität und/oder Selektivität dieser Materialien nicht den optimalen praktischen Anforderungen genügt.

Der Grund für diese unerwünschten Eigenschaften liegt darin, daß derartige Adsorbentien bevorzugt mittlere Porendurchmesser von beispielsweise 20 nm bis 1250 nm (EP 0 143 369) oder 1000 nm (DE 36 17 672) und/oder molekulare Ausschlußgrenzen für globuläre Proteine von 5 x 10⁴ bis 5 x 10⁶ Dalton (DE 39 26 539), größer 5 x 10⁵ Dalton (EP 0 424 698) und 1 x 10⁶ bis 1 x 10⁸ Dalton (EP 0 110 409 und EP 0 225 867) aufweisen, um bevorzugterweise und mit hoher Bindungskapazität biologische Makromoleküle, wie beispielsweise LDL mit einem Partikeldurchmesser von 20 - 30 nm und einem Molekulargewicht von 2,2 bis 3,5 x 10⁶ Dalton und/oder Fibrinogen mit einem Molekulargewicht von 3,4 x 10⁵ Dalton an der Poreninnenoberfläche zu adsorbieren.

Erfindungsgemäß werden daher zur selektiven und effektiven Elimination von TNF und LPS aus Körperflüssigkeiten wie Vollblut, Plasma und/oder Serum bevorzugt Trägermatrices verwendet, deren mittlerer Porendurchmesser kleiner 30 nm ist und/oder deren molekulare Ausschlußgrenze für globuläre Proteine kleiner 10⁶ Dalton, bevorzugterweise kleiner 2 x 10⁴ Dalton ist. Darüber hinaus werden erfindungsgemäß bevorzugt Trägermaterialien verwendet, deren immobilisierte Polyanionen ein mittleres Molekulargewicht von 600 bis 10⁶ Dalton, bevorzugterweise 5 x 10³ bis 5 x 10⁵ Dalton aufweisen. Bei der erfindungsgemäßen Verwendung derartig strukturierter Adsorbentien tritt keine unerwünschte Konkurrenzreaktion mit Makromolekülen wie LDL und/oder Fibrinogen um die polyanionischen Adsorptionszentren im Poreninneren auf (vergleichendes Beispiel 3).

TNF und LPS können daher in erwünschter Weise sehr selektiv und mit hoher spezifischer Bindungskapazität aus Körperflüssigkeiten in einem extracorporalen Perfusionssystem eliminiert werden.

Mit der erfindungsgemäßen Verwendung wird es außerdem ermöglicht, TNF und LPS in äußerst reiner Form zu erhalten, indem man die gebundenen Moleküle von der Säule nach an sich bekannten Methoden eluiert und zwar vorzugsweise mit steigenden Gradienten von Natriumchloridlösungen. Nach der Elution vom Adsorptionsmaterial kann die Konzentration des Tumor-Nekrose-Faktors oder LPS nach an sich bekannten Methoden bestimmt werden. Die erhaltenen Fraktionen können dann leicht so gesammelt werden, daß besonders reiner TNF und LPS erhalten wird.

In einer bevorzugten Ausführungsform der Erfindung wird bei Verwendung von Adsorptionsmaterialien mit einem mittleren Porendurchmesser größer 30 nm zur Herstellung von Plasma vor der nachfolgenden selektiven Elimination des TNF und LPS ein Plasmafraktionierungsfilter, der für Fibrinogen und/oder LDL impermeabel ist, verwendet, um eine Vorreinigung des Plasmas erreicht, die sich positiv auf das nachfolgende Reinigungs- bzw. Gewinnungsverfahren auswirkt. Diese beiden Plasmabestandteile könnten nämlich eventuell die Effektivität des erfindungsgemäßen Verfahrens beeinträchtigen. Die abgetrennten Bestandteile können sofort dem Patienten rückverabreicht werden.

Zur erfindungsgemäßen Verwendung kann eine Vorrichtung eingesetzt werden zur extracorporalen Entfernung des Tumor-Nekrose-Faktors und von Lipopolysacchariden aus wäßrigen Flüssigkeiten, insbesondere Blut, Plasma oder Serum, wobei diese Vorrichtung aus einem zylindrischen Gehäuse besteht, das mit einem Adsorptionsmaterial gefüllt ist, wie es erfindungsgemäß verwendet wird, und an seinen stirnseitigen Enden mit Deckeln versehen ist, die jeweils einen zentralen Zu- bzw. Ablaufstutzen aufweisen. Dieses zylindrische Gehäuse kann einen Durchmesser von 3 bis 20 cm, vorzugsweise 5 bis 10 cm und eine Länge von 1 bis 40 cm, vorzugsweise 5 bis 20 cm aufweisen. Das geeignete Meaterial für das Gehäuse ist Glas oder Kunststoff.

In die Deckel des zylindrischen Gehäuses können Siebe mit 10 bis 300 µm Porenweite, vorzugsweise 20 bis 100 µm Porenweite zur Eliminierung von Partikeln integriert sein. Die Vorrichtung ist in einer Verpackung mittels gamma-Strahlung oder Hitze sterilisierbar und damit besonders geeignet zum Einsatz in einem extracorporalen Perfusionssystem. Sie kann jedoch auch praktisch als Chromatrographiesäule, insbesondere zur erfindungsgemäßen Bestimmung der Konzentration des Tumor-Nekrose-Faktors und/oder zur erfindungsgemäßen präparativen Reinigung und Isolation des Tumor-Nekrose-Faktors verwendet werden.

Das Gehäusse der Vorrichtung kann in einen geschlossenen Kreislauf integriert sein, in dem die wäßrige Flüssigkeit mittels einer Pumpe zirkuliert wird. Hierbei kann sich im Kreislauf oder aber einer Zuleitung zum Kreislauf Plasmafraktionierungsfilter vorhanden sein, der für Fibrinogen und/oder LDL impermeabel ist. Hierdurch wird insbesondere bei Verwendung von Plasma eine Vorreinigung und Abtrennung von LDL und auch Albumin erreicht. Besonders geeignet ist eine Vorrichtung mit zwei zylindrischen Gehäusen (zwei Adsorberkapseln), die wechselseitig über Ventile angesteuert und mit der zu behandelnden wäßrigen Flüssigkeit in einem geschlossenen Kreislauf mittels einer Pumpe durchspült werden. Die jeweils nicht durchspülte, von dem Tumor-Nekrose-Faktor und/oder von LPS gesättigte Kapsel wird mit einer Regenerationslösung, vorzugsweise handelt es sich dabei um hochmolare Natriumchloridlösungen, eluiert. Für die Elution von LPS eignen sich auch wäßrige Lösungen von Amino-Verbindungen wie sie in EP 0 333 474 A2 beschrieben sind. Ein Membranfilter braucht, wenn überhaupt, hierbei natürlich nur einmal vorhanden sein, vorzugsweise in einer Zuleitung für die wäßrige Flüssigkeit. Ein weiterer Gegenstand der Erfindung ist ein Adsorptionsmaterial, bestehend aus einem porösen Trägermaterial mit daran kovalent gebundenen funktionellen Gruppen aus synthetischen oder/und halbsynthetischen oder/und natürlichen Polyanionenketten und zwar in linearer oder verzweigter Form bei dem der mittlere Porendurchmesser des Trägermaterials kleiner 30 nm und die molekulare Ausschlußgrenze für globuläre Proteine kleiner 2x10⁴ Dalton ist. Dieses Adsorptionsmaterial ist ganz besonders geeignet für die Durchführung die erfindungsgemäße Verwendung. Die folgenden Beispiele sollen in Verbindung mit den Abbildungen die Erfindung weiter erläutern.

### Beispiel 1

### Eliminierung des TNF aus Humanplasma

Adsorbens: Dextransulfatcellulose (Liposorber™ LA-15, Kanegafuchi Chemical Industry, Osaka, Japan)

### Versuchsdurchführung:

600 ml frisch gewonnenes Humanplasma wurden mit Tumor-Nekrose-Faktor (TNFα, Hofmann La-Roche, Basel) versetzt, so daß die Konzentration an TNF 1410 pg/ml beträgt.

Anschließend wurde das Humanplasma mit einer Flußrate von 10 ml/min über die mit einer Lösung (Ringerlösung bestehend aus NaCl (140 mmol/l), CaCl₂ (2 mmol/l) und KCl (4 mmol/l), pH 6,6, äquilibrierte Adsorberkapsel, deren Bettvolumen ca. 150 ml beträgt, mit Hilfe einer Schlauchpumpe gepumpt.

Die ersten 50 ml des Kapseleluats wurden verworfen (Verdünnungseffekte durch Kapseltotvolumen). In den restlichen, jeweils 50 ml-Eluatfraktionen erfolgte die Bestimmung des TNF.

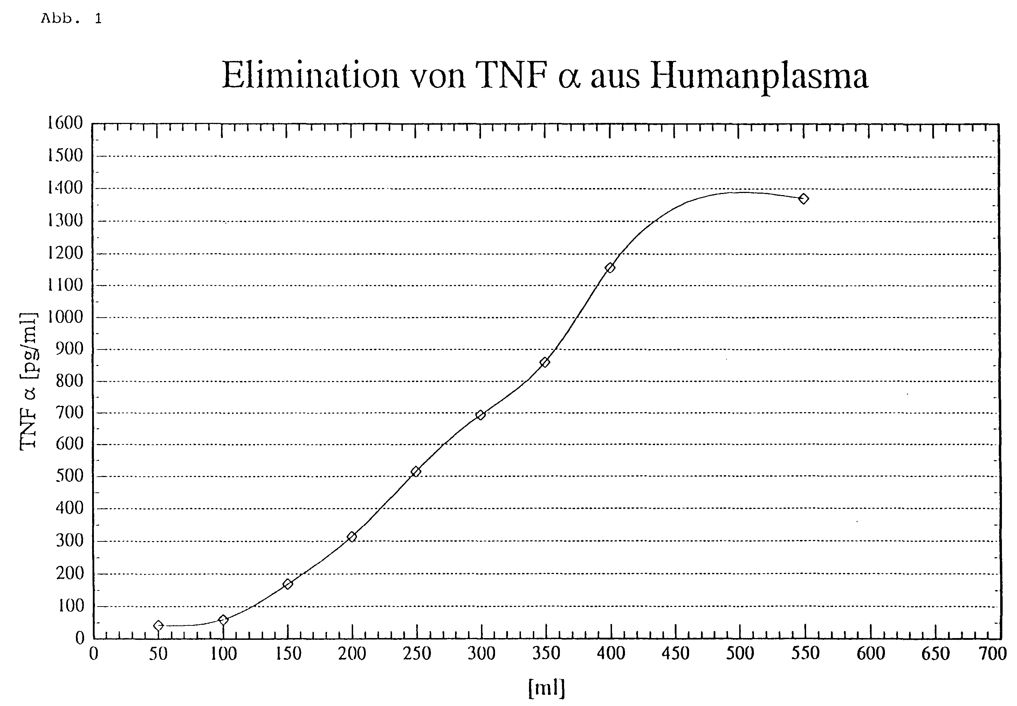

Der Verlauf der Adsorptionskurve in Abbildung 1 zeigt, daß die erfindungsgemäß verwendete Dextransulfatcellulose den Tumor-Nekrose-Faktor vollständig aus dem behandelten Humanplasma eliminiert.

### Beispiel 2

### Eliminierung von Lipopolysacchariden (LPS) aus Humanplasma

Adsorbens: Dextransulfatcellulose (Liposorber ™ LA-15, Kanegafuchi Chemical Industry, Osaka, Japan)

### Versuchsdurchführung:

10 ml frisch gewonnenes Humanplasma wurden mit 1200 pg E.coli Endotoxin Serotyp 0111:B4 (LPS) versetzt. Eine Einmal-Säule (5 x 70 mm) wurde mit der Dextransulfatcellulose gepackt (Säulenbettvolumen: 3 ml), mit Ringerlösung äquilibriert und hitzesterilisiert (121°C, 30 min). Unter sterilen Bedingungen wurde das LPS-Plasma über die Säule gegeben und im Eluat die Menge an LPS mit dem quantitativen Limulus-Abmöbozytenlysat-Test (QCL-1000, Fa. Whittaker, Walkersville, MD) bestimmt.

### Versuchsergebnis:

Der quantitative Endotoxin-Test im Säuleneluat ergab einen Wert von 850 pg LPS. Ca. 30 % der zugesetzten LPS-Menge werden somit von der erfindungsgemäß verwendeten Dextransulfatcellulose adsorbiert bzw. eliminiert.

### Beispiel 3

Vergleichende Untersuchung zur Bindungskapazität und Bindungsselektivität für den Tumor-Nekrose-Faktor (TNF) aus Humanserum.

### Adsorbentien:

I) Fractogel TSK HW 40 C (Bezugsquelle: E. Merck, Darmstadt) mit einem Fraktionierungsbereich für globuläre Proteine von 10² bis 10⁴ Dalton, einer Teilchengröße von 50 bis 100 µm und einem mittleren Porendurchmesser von 12 nm wurde in bekannter Weise mit Epichlorhydrin (1-Chlor-2,3-epoxypropan) aktiviert und anschließend mit Dextransulfat (Molekulargewicht: 5 x 10³ Dalton) umgesetzt.
II) Fractogel TSK HW 65 M (Bezugsquelle: E. Merck, Darmstadt) mit einem Fraktionierungsbereich für globuläre Proteine von 5 x 10⁴ bis 5 x 10⁶ Dalton, einer Teilchengröße von 45 bis 90 µm und einem mittleren Porendurchmesser von 100 nm wurde in bekannter Weise mit Epichlorhydrin (1-Chlor-2,3-epoxypropan) aktiviert und anschließend mit Dextransulfat (Molekulargewicht: 5 x 10³ Dalton) umgesetzt.

### Versuchsdurchführung:

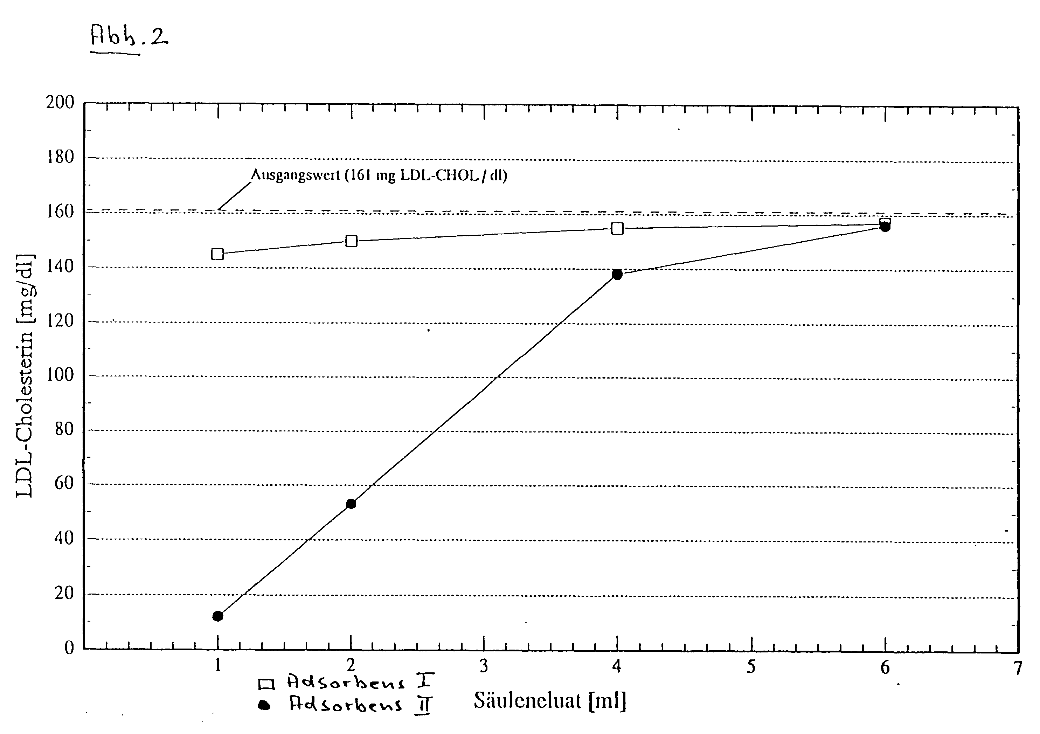

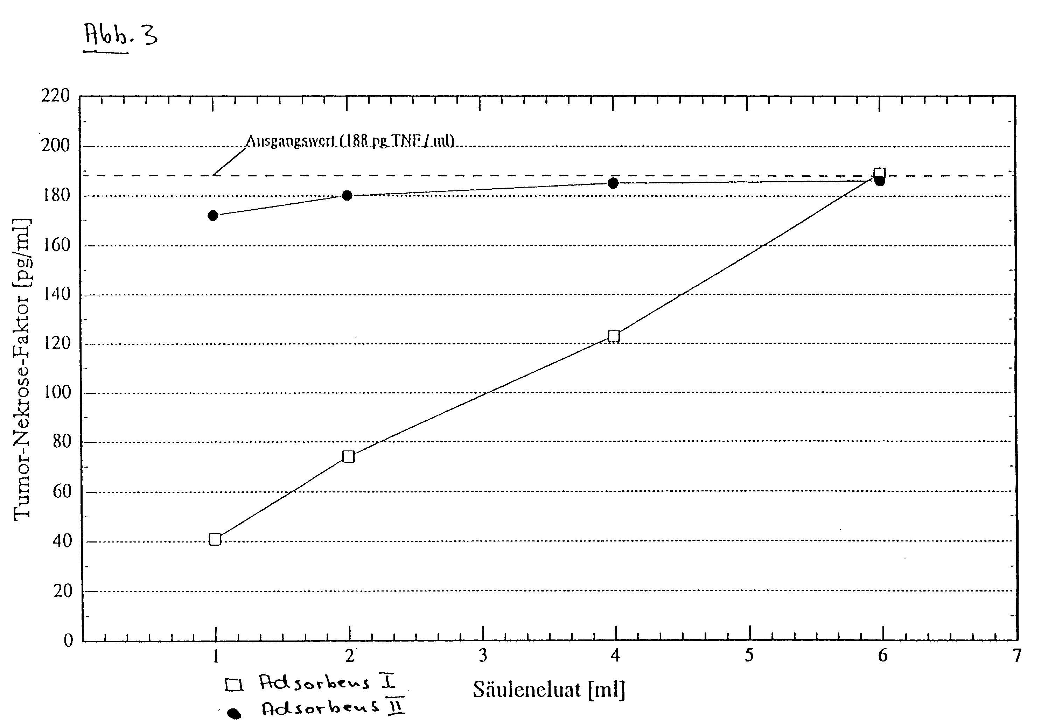

6 ml frisch gewonnenes Humanserum wurden mit Tumor-Nekrose-Faktor (TNF, Hofmann La-Roche, Basel) versetzt, so daß die Konzentration an TNF 188 pg/ml betrug. Anschließend wurde diese Humanserumprobe bei Raumtemperatur über eine mit dem Adsorbens I oder II gefüllte und mit einer Lösung (Ringer-Lösung, pH 6,6) bestehend aus NaCl (140 mmol/l) CaCl₂ (2 mmol/l) und KCl (4 mmol/l) äquilibrierte Säule (5 x 80 mm; Säulenbettvolumen: 2 ml) gegeben und im Säuleneluat (1 ml Fraktionen) die Konzentration an Tumor-Nekrose-Faktor (TNF) und Low-Density-Lipoprotein Cholesterin (LDL-Cholesterin) bestimmt. Die Werte der TNF und der LDL-Cholesterin Bestimmung wurden in Abhängigkeit des Eluat-Volumens aufgetragen (Abb. 2 und Abb. 3).

Der Verlauf der Adsorptionskurven für TNF und LDL-Cholesterin in Abb. 2 und Abb. 3 zeigt, daß nur das erfindungsgemäße Adsorbens I in erwünschter Weise sehr effizient den Tumor-Nekrose-Faktor bindet und nur geringe Menge an Low-Density-Lipoproteinen (LDL-Cholesterin) adsorbiert, während das Adsorbens II nahezu ausschließlich nur die Low-Density-Lipoproteine (LDL-Cholesterin) eliminiert.

## Patentansprüche

1. Verwendung eines Adsorptionsmaterials aus einem porösen Trägermaterial mit einem mittleren Porendurchmesser von kleiner 30 nm oder/und mit einer molekularen Ausschlußgrenze für globuläre Proteine von kleiner 10⁶, insbesondere von kleiner 2 x 10⁴ Dalton mit daran kovalent gebundenen funktionellen Gruppen aus synthetischen oder/und halbsynthetischen oder/und natürlichen Polyanionenketten mit einem mittleren Molekulargewicht von 600 bis 10⁶ Dalton, insbesondere 5 x 10³ bis 5 x 10⁵ Dalton, und zwar in linearer oder verzweigter Form zur Herstellung eines Arzneimittels zur Behandlung eines septischen Schockes unter quantitativer, selektiver Entfernung von Tumor-Nekrose-Faktor (TNF) und Lipopolysacchariden aus Blut, Plasma oder Serum.

2. Verwendung nach Anspruch 1,
**dadurch gekennzeichnet,**
**daß** die natürlichen Polyanionenketten aus biologischen Polycarbonoder/und Polysulfonsäuren und insbesondere aus sulfatierten Polysacchariden bestehen.

3. Verwendung nach Anspruch 1 oder 2,
**dadurch gekennzeichnet,**
**daß** die synthetischen bzw. halbsynthetischen Ketten Polymerisate oder Copolymerisate der MonomereAcrylsäure, Methacrylsäure, Vinylsulfonsäure, Maleinsäure; Acrylsäure- oder/und Methacrylsäurederivate der Formel H₂C = CR₁ - CO - R₂, wobei der Substituent R₁ Wasserstoff oder eine Methylgruppe und R₂ eine amid- oder esterartig gebundene lineare oder/und verzweigtkettige aliphatische Sulfonsäure-, Carbonsäureoder/und Phosphorsäuregruppe ist; Styrolsulfonsäure, Anetholsulfonsäure, Styrolphosphorsäure; Glutaminsäure, Asparaginsäure; Adenosin-3',5'-diphosphat, Guanosin-3',5'-diphosphat sind.

4. Verwendung nach einem der vorhergehenden Ansprüche,
**dadurch gekennzeichnet,**
**daß** das Trägermaterial aus porösem Glas, porösem oder/und mit organischen Polymerisaten oder Copolymerisaten beschichtetem Kieselgel, vernetzten Kohlehydraten oder/und organischen Polymerisaten oder Copolymerisaten besteht.

5. Verwendung nach einem der vorhergehenden Ansprüche,
**dadurch gekennzeichnet,**
**daß** das Trägermaterial aus Cellulose besteht und als Polyanionenkette Dextransulfat vorhanden ist.

6. Verwendung nach einem der vorhergehenden Ansprüche,
**dadurch gekennzeichnet,**
**daß** das Mittel in einem extrakorporalen Perfusionssystem eingesetzt wird.

## Claims

1. Use of an adsorption material which consists of a porous supporting material having an average pore diameter of less than 30 nm and/or a molecular exclusion limit for globular proteins of less than 10⁶, in particular of less than 2 x 10⁴ daltons, to which functional groups consisting of synthetic and/or semisynthetic and/or natural polyanion chains with an average molecular weight of 600 to 10⁶ daltons, in particular 5 x 10³ to 5 x 10⁵ daltons, are covalently bound, namely in a linear or branched form, for the preparation of a medicament for the treatment of septic trauma with quantitative selective removal of tumour necrosis factor(TNF) and lipopolysaccharides from blood, plasma or serum.

2. Use according to Claim 1, **characterised in that** the natural polyanion chains consist of biologial polycarboxylic acids and/or polysulphonic acids and, in particular, of sulphated polysaccharides.

3. Use according to Claim 1 or 2, **characterised in that** the synthetic or semisynthetic chains are polymers or copolymers of the monomers acrylic acid, methacrylic acid, vinyl sulphonic acid, maleic acid; acrylic acid derivatives and/or methacrylic acid derivatives having the formula H₂C = CR₁ - CO - R₂, wherein the substituent R₁ is hydrogen or a methyl group and R₂ is an amidically or esterically linked linear and/or branched-chain aliphatic sulphonic acid, carboxylic acid and/or phosphoric acid group; styrene sulphonic acid, anethol sulphonic acid, styrene phosphoric acid; glutamic acid, aspartic acid; adenosine-3',5'-diphosphate, guanosine-3',5'-diphosphate.

4. Use according to any one of the preceding Claims, **characterised in that** the supporting material consists of porous glass, porous silica gel and/or silica gel coated with organic polymers or copolymers, cross-linked carbohydrates and/or organic polymers or copolymers.

5. Use according to any one of the preceding Claims, **characterised in that** the supporting material consists of cellulose and dextran sulphate is provided as the polyanion chain.

6. Use according to any one of the preceding Claims, **characterised in that** the medicament is used in an extracorporeal perfusion system.

## Revendications

1. Utilisation d'un matériau adsorbant constitué d'un matériau support poreux ayant un diamètre de pores moyen inférieur à 30 nm et/ou un seuil de coupure moléculaire pour des protéines globulaires inférieur à 10⁶, en particulier inférieur à 2 × 10⁴ daltons, auquel sont liés par covalence des groupes fonctionnels de chaînes polyanioniques synthétiques et/ou semi-synthétiques et/ou naturelles ayant une masse molaire moyenne de 600 à 10⁶ daltons, en particulier de 5 × 10³ à 5 × 10⁵ daltons, sous forme linéaire ou ramifiée, pour la préparation d'un médicament destiné au traitement du choc septique par élimination quantitative et sélective du facteur de nécrose tumorale (TNF) et des lipopolysaccharides du sang, du plasma ou du sérum.

2. Utilisation selon la revendication 1, **caractérisée en ce que** les chaînes polyanioniques naturelles sont constituées de poly(acides carboxyliques) et/ou de poly(acides sulfoniques) biologiques et en particulier de polysaccharides sulfatés.

3. Utilisation selon la revendication 1 ou 2, **caractérisée en ce que** les chaînes synthétiques ou semi-synthétiques sont des polymères ou des copolymères des monomères acide acrylique, acide méthacrylique, acide vinylsulfonique, acide maléique; de dérivés d'acide acrylique et/ou d'acide méthacrylique de formule H₂C=CR₁-CO-R₂, dans laquelle le substituant R₁ représente l'hydrogène ou un groupe méthyle et R₂ est un groupe acide sulfonique, acide carboxylique et/ou acide phosphorique aliphatique linéaire et/ou ramifié lié par une liaison de type amide ou ester; de l'acide styrènesulfonique, de l'acide anétholsulfonique, de l'acide styrènephosphorique; de l'acide glutamique, de l'acide aspartique; de l'adénosine-3',5'-diphosphate, du guanosine-3',5'-diphosphate.

4. Utilisation selon l'une des revendications précédentes, **caractérisée en ce que** le matériau support est constitué de verre poreux, de gel de silice poreux et/ou revêtu de polymères ou de copolymères organiques, d'hydrates de carbone réticulés et/ou de polymères ou copolymères organiques.

5. Utilisation selon l'une des revendications précédentes, **caractérisée en ce que** le matériau support est constitué de cellulose et **en ce que** du sulfate de dextrane est présent en tant que chaîne polyanionique.

6. Utilisation selon l'une des revendications précédentes, **caractérisée en ce que** le médicament est utilisé dans un système de perfusion extracorporelle.
